# EUROPEAN PATENT SPECIFICATION

(11) **EP 0 620 737 B1**
(45) Date of publication and mention of the grant of the patent: **20.08.1997**
(21) Application number: 93901738.0
(22) Date of filing: 06.01.1993
(51) Int. Cl.: A61K 38/00, A61K 38/55

(54) **Use of LACTOFERRIN iN the manufacture of a medicament for treating RHEUMATISM**
Verwendung von LACTOFERRIN zur Herstellung eines THERAPEUTISCHEN MITTELS zur Behandlung von RHEUMATISMUS
Utilisation DE LA LACTOFERRINE pour la fabrication d'un médicament destiné au traitement du RHUMATISME

(30) Priority: 08.01.1992 JP 1648/92
(43) Date of publication of application: 26.10.1994
(73) Proprietor: BIO SERAE LABORATOIRES SA, 12400 Saint Affrique (FR)
(72) Inventor: HIKIDA, Mitsushi 1-417, 15, Kitaoohi-cho, Osaka (JP); HAYASHI, Masaaki 1-15, Iguchido 3-chome, Osaka (JP); DEGRE, Michel, François, F-12400 Saint-Affrique (FR)
(74) Representative: Cabinet BARRE LAFORGUE & associés
(86) International application number: EP9300015
(87) International publication number: WO9313790

(56) References cited:
- WO-A-86/04217
- DATABASE WPIL Section Ch, Week 8927, Derwent Publications Ltd., London, GB; Class D21, AN 89-19715; & JP-A-1 135 726
- DATABASE WPIL Section Ch, Week 8749, Derwent Publications Ltd., London, GB; Class D21, AN 87-343748
- DATABASE WPIL Section Ch, Week 9036, Derwent Publications Ltd., London, GB; Class D21, AN 90-269969
- DATABASE WPIL Section Ch, Week 9120, Derwent Publications Ltd., London, GB; Class B04, AN 91-142204
- THE SCANDINAVIAN JOURNAL OF CLINICAL AND LABORATORY INVESTIGATION, vol. 46, no. 7, 1986, OSLO, NORWAY, pages 695-704; BAYNES ET AL: 'THE NON-IMMUNE INFLAMMATORY RESPONE: SERIAL CHANGES IN PLASMA IRON, IRON-BINDING CAPACITY, LACTOFERRIN, FERRITIN AND C-REACTIVE PROTEIN'
- CLINICAL AND EXPERIMENTAL RHEUMATOLOGY, vol. 8, 1990, PISA, ITALY, pages 159-162; JENSEN ET AL: 'RELEASE OF ELASTOLYTIC ACTIVITY FROM HUMAN MONOCYTES AND GRANULOCYTES IN VITRO BY IMMUNE COMPLEX STIMULATION'
- ARTHRITIS AND RHEUMATISM, vol. 27, no. 4, 1984, NEW YORK, US, pages 462-467; KONTTINEN ET AL: 'LACTOFERRIN IN SJOGREN'S SYNDROME'
- JOURNAL OF ENZYME INHIBITION, vol. 2, no. 1, 1987, LONDON, GB, pages 1-22; JOHNSON ET AL: "COOLGENASE INHIBITORS: THEIR DESIGN AND POTENTIAL THERAPEUTIC USE"

## Description

This invention relates to the use of lactoferrin for the manufacture of a medical preparation for the treatment of rheumatism.

Lactoferrin, a protein existing in milk or tears of human being, bovine, etc., is known to have pharmacological effects such as antibacterial effect and proliferating effect of lymphocytes (JP-48534/1990, JP-A-1135726, FR-A-2651433). Lactoferrin is also known to act against free radical (FR-A-2596986, FR-A-2641696, Arthritis and rheumatism, vol. 27, n° 4, 1984, New York, U.S.A., p. 462-467).

However, it is desired to study potential effects of lactoferrin, a natural product, and expand the applications thereof.

Thereupon, we studied to find new pharmacological effects of lactoferrin and found that lactoferrin was useful for treatment of rheumatism. It is noted that the two following documents study the effects of lactoferrin in inflammatory joint diseace : Phil. Trans. R. Soc. Lon. (1985) B 311, p. 659-71 ; Scandinavian J. of Chemical Investigations (1986), vol. 46, p. 695-704. However, no clear conclusion can be drawn from these documents on the effective effect of lactoferrin on this kind of diseace.

It is expected that lactoferrin, a natural protein, has a possibility to be applied for treatment of various diseases. However, as the pharmacological effects of lactoferrin, only antibacterial effect and proliferating effect of lymphocytes etc. have been reported. Therefore, we studied to find new pharmacological effects of lactoferrin and found that lactoferrin was useful for treatment of rheumatism.

An inhibitory effect of a medical substance against collagenase activity can be used as an index to examine the utility thereof for antirheumatic agent. The relationship of inhibitory effect against collagenase activity and rheumatism has been reported (W. H. Johnson et. al., J. Enzyme Inhibition, 2, 1 (1987) ; Woolley D. E. et. al., Arthritis Rheum., 20, 1231 (1977)). Therefore, we examined the inhibitory effect of lactoferrin against collagenase activity.

As the result, we found that lactoferrin significantly inhibited the degradation of collagen caused by collagenase and showed excellent inhibitory effect against collagenase activity. The detailed experimental data are shown in the article of pharmacological test.

Thus, this invention proposes to use lactoferrin for the manufacture of a composition for inhibit the collagenable activity in or on tissues containing collagenase enzyme, the composition containing the lactoferrin in concentrations between 0.005 % and 5 %, preferably between 0.05% and 0.5 %. In particular, lactoferrin can be used for the manufacture of a medical preparation for treatment of rheumatism.

Lactoferrin is usually administered directly to knee joint etc. by injection, but it may be administered orally. The dosage is adjusted depending on symptom, dosage form etc. In case of injection, the usual concentration of lactoferrin is 0.1% - 10%, preferably 0.5% - 5%, and suitable volume of the solution is injected to a rheumatoid joint. In case of oral form, the usual daily dosage is 1 to 5000 mg, preferably 50 - 1000 mg in one or a few divided doses.

The preparations of lactoferrin can be prepared by usual methods. Injection can be prepared by dissolving lactoferrin in distilled water for injection and, if necessary, usual excipients such as isotonic agent, pH adjusting agent and viscosity agent can be added. Tablet can be prepared by using usual excipients such as binding agent and lubricant.

Examples of formulations are shown below.

### Examples of Formulations

### 1) Injection

| formulation 1 | |
|---|---|
| lactoferrin | 1g |
| sodium chloride | 0.9g |
| distilled water for injection | q.s. |
| total | 100ml |

| formulation 2 | |
|---|---|
| lactoferrin | 2g |
| sodium chloride | 0.9g |
| distilled water for injection | q.s. |
| total | 100ml |

| formulation 3 | |
|---|---|
| lactoferrin | 5g |
| sodium chloride | 0.7g |
| distilled water for injection | q.s. |
| total | 100ml |

| formulation 4 | |
|---|---|
| lactoferrin | 0.5g |
| sodium chloride | 0.9g |
| distilled water for injection | q.s. |
| total | 100ml |

| formulation 5 | |
|---|---|
| lactoferrin | 10g |
| sodium chloride | 0.5g |
| distilled water for injection | q.s. |
| total | 100ml |

| formulation 6 | |
|---|---|
| lactoferrin | 0.1g |
| sodium chloride | 0.9g |
| methyl cellulose | 0.5g |
| distilled water for injection | q.s. |
| total | 100ml |

### 2) Tablet

| formulation 7 | |
|---|---|
| lactoferrin | 100mg |
| lactose | 50mg |
| crystalline cellulose | 30mg |
| hydroxypropylcellulose | 5mg |
| magnesium stearate | 5mg |
| total | 190mg |

### PHARMACOLOGICAL TEST

We examined the inhibitory effect of lactoferrin against collagenase activity according to the method of Nagai et. al. (Japanese Journal of Inflammation, 4, 123 (1984)).

### Inhibitory effect against collagenase derived from microorgan (Experimental Method)

Collagen labeled with fluorescein isothiocyanate and collagenase derived from microorgan (Clostridium histolyticum) were dissolved in Tris-HCl buffer (0.05M, pH7.5), which contains sodium chloride (0.2M), calcium chloride (0.005M) and sodium azide (0.02%), in a concentration of 0.025% and 12.5 unit/ml respectively. To 0.4ml of this solution, lactoferrin was added and the mixture was incubated for 1hr at 35°C in a brown test tube. 10µl of water-ethanol solution (1:1 v/v) dissolving o-phenanthroline (80mM) was added to stop the reaction and the mixture was incubated for 1hr at 35°C. 400µl of a mixture of Tris-HCl buffer (0.05M, pH9.5), which contains sodium chloride (0.2M), and ethanol (3:7 V/V) was added and the mixture was stirred and centrifuged. Degradated collagen was assayed by measuring the fluorescence intensity of the supernatant (excitation wavelength : 495 nm, emission wavelength : 520nm).

In the control, it was treated by the same manner as the above except the addition of lactoferrin.

### (Result)

Inhibitory percentage against the degradation of collagen by the addition of lactoferrin was shown in Table 1.

**Table 1**

| | | fluorescence intensity | inhibition(%) |
|---|---|---|---|
| control | | 42.6 ± 0.7 | |
| lactoferrin | 0.3125mg/ml | 38.0 ± 0.8 | 10.6 |
| | 0.625 | 33.7 ± 0.5 | 20.9 |
| | 1.25 | 24.6 ± 0.9 | 42.3 |
| | 2.5 | 15.7 ± 1.1 | 63.1 |
| | 5.0 | 7.5 ± 0.9 | 82.4 |
| | 10.0 | 2.5 ± 0.4 | 94.1 |

### Inhibitory effect against collagenase derived from tissue (Experimental Method)

The inhibitory effect against tissue collagenase, which was purified from cornea of rabbit according to the method reported by Burns et al. (Invest. Ophthalmol. Vis. Sci., 30, 1569 (1989)), was examined by the similar method as for collagenase derived from microorgan. In this experiment, the amount of collagenase to be added was 2.5mg protein/ml.

### (Result)

The experimental results were shown in Table 2.

**Table 2**

| | | fluorescence intensity | inhibition(%) |
|---|---|---|---|
| control | | 34.7 ± 1.7 | |
| lactoferrin | 5.0mg/ml | 28.0 ± 2.8 | 19.3 |
| | 10.0 | 14.4 ± 1.7 | 58.5 |

As shown in Tables 1 and 2, we found that lactoferrin inhibited the degradation of collagen in a dose-dependent manner and showed an inhibitory effect against collagenase activity in the both experiments using collagenase derived from microorgan or tissue.

As shown in the article of pharmacological test, lactoferrin shows excellent inhibitory effect against collagenase activity and is useful for treatment for rheumatism.

As it was shown that lactoferrin is able to inhibit bacterial and tissue collagenase activity in a dose dependent manner, it is evident that this activity is of interest to cosmetic application.

Skin ageing is a complex process, but it is well known that enzymatic hydrolysis of tissue specific macromolecules, such as collagen and elastine, can contribute to premature scenescence. Natural, inoffensive and efficacious collagenase inhibitors are therefore of interest. Lactoferrin can therefore be used as an agent for the prevention of premature ageing due to its contribution to the preservation of the integrity of the skin.

The concentrations at which lactoferrin is active against these collagenases are of reasonable range for dermatology and cosmetic applications.

Cosmetic applications include oral hygiene also, where lactoferrin may be of use in the inhibition of bacterial collagenases : prevention of bacteriolitic destruction of the gums.

Therefore, in dermatological and cosmetic compositions, lactoferrin is used as inhibitor of bacterial and tissues collagenases, in concentrations between 0.005 % and 5 % (preferably between 0.05 % and 0.5 %).

Lactoferrin may also be used for dermatological and cosmetic compositions in the form of creams, lotions, milks, tooth paste, mouthwash rinses, gels, for application in skin care, oral hygiene and prevention of tissue degradation.

## Claims

1. Use of lactoferrin for the manufacture of a medical preparation for the treatment of rheumatism.

2. Use of lactoferrin for the manufacture of a composition for inhibiting the collagenase activity in or on tissues containing collagenase enzyme, the composition containing the lactoferrin in concentrations between 0.005 % and 5 %, preferably between 0.05 % and 0.5 %.

## Patentansprüche

1. Verwendung von Lactoferrin zur Herstellung eines therapeutischen Mittels zur Behandlung von Rheumatismus.

2. Verwendung von Lactoferrin zur Herstellung eines zur Hemmung der Collaginase-Aktivität in bzw. auf das Enzym Collaginase enthaltenden Geweben bestimmten Präparats, wobei das besagte Präparat das Lactoferrin in Konzentrationen zwischen 0,005 % und 5 % aber vorzugsweise zwischen 0,05 % und 0,5 % enthält.

## Revendications

1. Utilisation de la lactoferrine pour la fabrication d'un médicament destiné au traitement des rhumatismes.

2. Utilisation de la lactoferrine pour la fabrication d'une composition pour inhiber l'activité de la collagénase dans ou sur des tissus contenant l'enzyme collagénase, la composition contenant la lactoferrine en concentrations de 0,005% à 5%, de préférence de 0,05% à 0,5%.
